(19)

Europäisches
Patentamt
European
Patent Office
Office européen
des brevets

(11)  **EP 1 871 467 B1**

(12)  **EUROPEAN PATENT SPECIFICATION**

(45) Date of publication and mention
of the grant of the patent:
**27.07.2011  Bulletin 2011/30**

(51) Int Cl.:
*A61N 1/36* (2006.01)       *A61N 1/04* (2006.01)
*A61N 1/32* (2006.01)

(21) Application number: **06744722.7**

(22) Date of filing: **23.03.2006**

(86) International application number:
**PCT/IB2006/001298**

(87) International publication number:
**WO 2006/100609 (28.09.2006 Gazette 2006/39)**

(54) **NERVE STIMULATION APPARATUS**

VORRICHTUNG ZUR NERVENSTIMULATION

APPAREIL DE STIMULATION NERVEUSE

(84) Designated Contracting States:
**AT BE BG CH CY CZ DE DK EE ES FI FR GB GR
HU IE IS IT LI LT LU LV MC NL PL PT RO SE SI
SK TR**

(30) Priority:  **23.03.2005  GB 0505940**

(43) Date of publication of application:
**02.01.2008  Bulletin 2008/01**

(73) Proprietor: **BMR Research and Development
Limited
Galway (IE)**

(72) Inventor: **CONOR, Minogue
Kinvara,
Co. Calway (IE)**

(74) Representative: **Murnane, Graham John et al
Murgitroyd & Company
165-169 Scotland Street
Glasgow
G5 8PL (GB)**

(56) References cited:
**GB-A- 2 374 288      US-A- 4 541 417
US-A- 4 729 377      US-A1- 2002 058 972**

EP 1 871 467 B1

**Description**

The present invention relates to nerve stimulation apparatus, and more particularly but not exclusively, relates to nerve stimulation apparatus for stimulating muscles by way of the muscle motor nerve in order to maximise the benefit obtained by muscle stimulation, for example whilst exercising.

Individuals are generally interested in obtaining maximum benefit from a given amount of exertion when exercising. This can currently be facilitated using Electrical Muscle Stimulation (EMS) devices which pass a controlled electrical current through the motor nerve of targeted muscles (such as the abdominal muscles) in order to cause those muscles to contract. This has the effect of toning those targeted muscles with minimal effort and can be used whilst performing an exercise routine in order to maximise benefit from the routine. Although EMS devices assist toning of muscles they do not provide any way of ensuring that cardiovascular exercise is optimised.

For example, the applicant's earlier UK Patent Application No. GB2374288A discloses apparatus for attachment to a user's legs for inducing a shivering phenomenon therein. Use of a separate heart rate monitor is briefly described only in the context of being a safety feature to prevent "overstressing" of the user.

According to the present invention there is provided nerve stimulation apparatus according to claim 1.

embodiments of the present invention will now be described, by way of example only, with reference to the accompanying figures, in which:-

　　Fig. 1 is a schematic illustration of apparatus in accordance with the present invention as worn by a user during exercise (with sections cut away for clarity);
　　Fig. 2 is a schematic block diagram showing the components of the apparatus of Fig. 1; and
　　Fig. 3 is a schematic block diagram showing the components of the apparatus of Fig. 1 with additional protective circuits installed; and
　　Fig. 4 is a more detailed illustration of the apparatus of Fig. 1 prior to use.

It should be noted that for the sake of clarity, the following description will refer to the location of components from the user's point of view when in use, that is components described as being on the left hand side of the apparatus relate to those components provided on the user's left hand side and components described as being on the user's right hand side relate to those components on the user's right hand side in use.

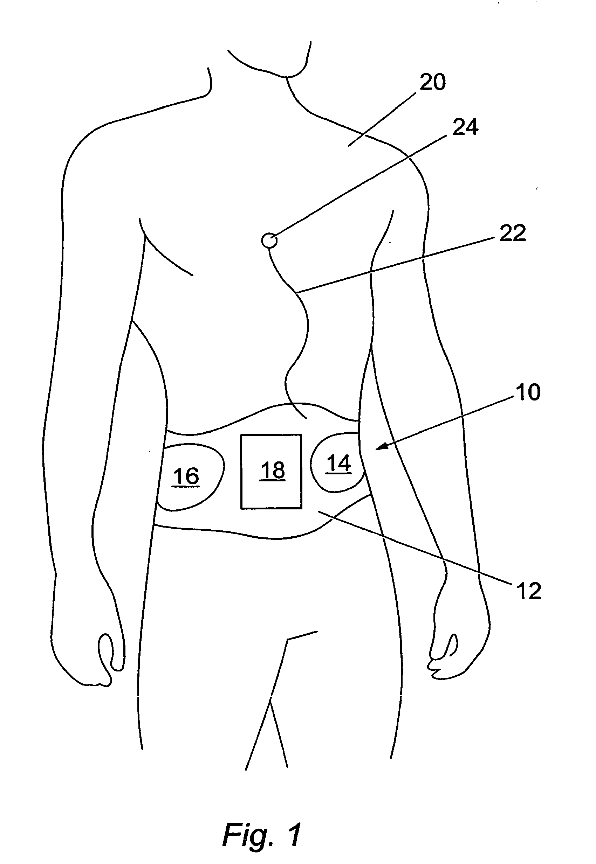

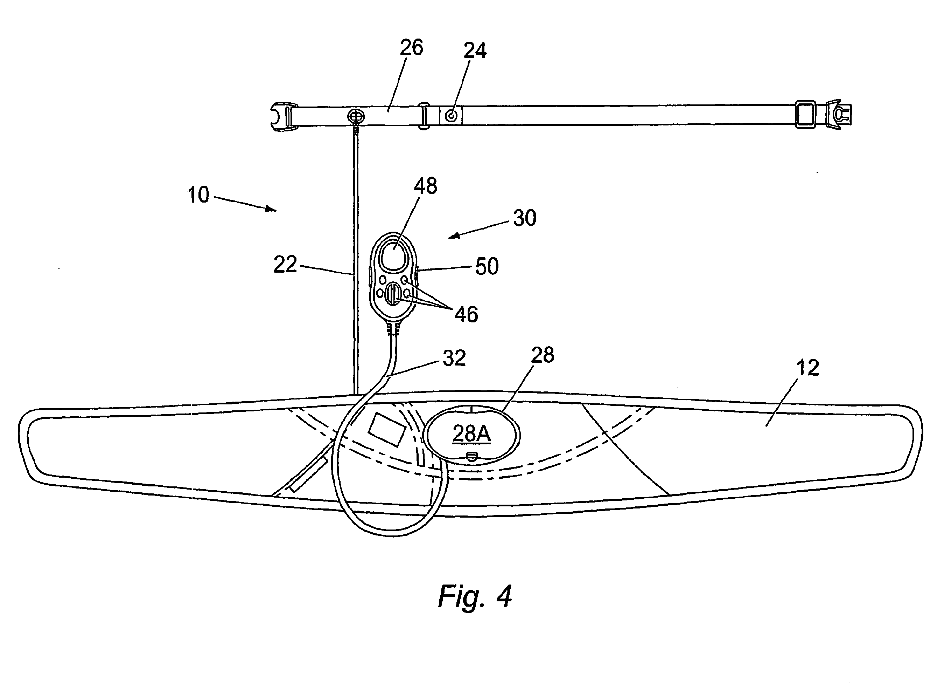

Referring to Figs. 1 and 4, the apparatus 10 comprises an abdominal belt 12 having left electrode 14, right electrode 16 and central electrode 18 mounted on the inner circumference thereof such that the electrodes 14, 16 and 18 make contact with the user's 20 abdomen.

A remote sensor 24 is also provided on the user's chest by a suitable attachment means such as an adjustable chest strap 26 (for a male user) as shown in Fig. 4 or a re-usable patch, not shown,(for a female user). The remote sensor 24 is connected, via a lead 22, to a base unit 28 on the belt 12. The base unit 28 is provided with a control unit 30 which may be remote from the base unit and connected via a control lead 32.

The abdominal belt 12 is shaped to provide a comfortable fit around the abdomen of the user 20-when in use (for both male and female users) and may be made of any suitable material such as neoprene or other elastic material. The abdominal belt 12 is provided with a docking location which locates the base unit 28 on the belt 12.

The left electrode 14 and right electrode 16 are of typical construction and are each positioned along the band 12 in a position which ensures that when the band 12 is secured around the abdomen of the user 20, the electrodes 14 and 16 will contact the area of skin between the user's rib cage and pelvis on the left and right hand side of the user. The correct location of electrodes 14 and 16 is important since the abdominal muscle motor nerves are confined between the rib cage and the pelvis.

The central electrode 18 is located at the centre of the belt 12 such that when the belt 12 is attached to the abdomen the electrode 18 will contact the skin at the umbilicus. This allows further targeted stimulation of the central abdominal muscles. The central electrode 18 is similar in composition to the left and right electrodes 14 and 16 and provides a common node for the current from the left electrode 14 to pass when the left abdominal muscles are stimulated and for the current from the right electrode 16 to pass when the right abdominal muscles are stimulated. It will be understood by the skilled reader that in the following description muscles are actually stimulated by stimulating the motor nerve which supplies them.

Selected electrodes of the group of electrodes 14, 16 and 18 may be capable of detecting electrical signals in addition to producing electrical current, as will be discussed subsequently.

Remote sensor 24 comprises a sensor capable of detecting electrical signals produced by a heart beat and is used in conjunction with at least one of the group of electrodes 14, 16 and 18 for this purpose.

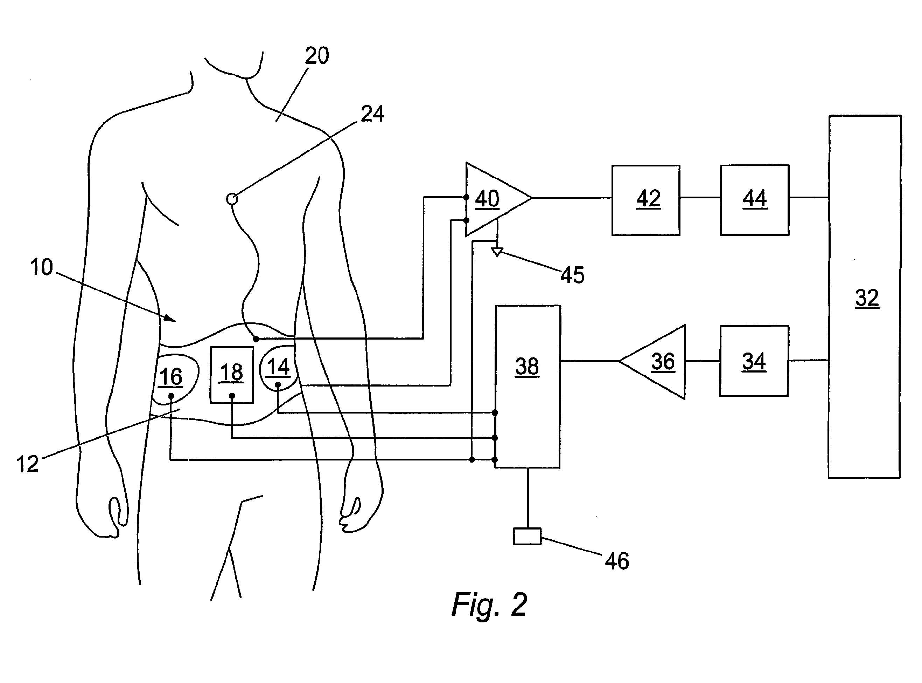

The base unit 28 comprises a body 28A which houses the electrical components required to control the system. Referring to Fig. 2, the electrical components include a micro-controller 32, digital to analogue converter 34, pulse amplifier 36, current steering network 38, differential amplifier 40, band pass filter 42 and threshold detector 44. The interaction between these components will be described subsequently.

Control unit 30 is provided with control buttons 46 which allow the user to vary the intensity of the muscle stimulation provided by the apparatus 10 and to switch between operation modes etc. A display screen 48 allows the user to view information such as the intensity, duration of the exercise, and Quality Factor (discussed subsequently). A clip 50 is also provided on the rear of the unit 30 in order to allow the user to clip the base unit to the belt 12 (or other location) as desired. This frees the user's hands for use in the exercise being performed.

In use, the belt 12 of the apparatus 10 is attached to the user's abdomen such that electrodes 14, 16 and 18 on the belt contact the skin at the locations previously described. The remote sensor 24 is then attached to the chest via the chest strap 26 (for a male user) or the patch (for a female user). If not already in place, the base unit 28 and control unit 30 are then attached to the belt 12. Once switched on, the apparatus 10 is ready for use.

When the user begins to exercise he/she can select a level of nerve (and hence muscle) stimulation using the control unit 30. Depending upon the mode and level of muscle stimulation selected, the micro-controller 32 initiates stimulation signals through the digital to analogue converter 34. Each analogue pulse of the signal from the converter 34 is amplified by pulse amplifier 36 which inputs the amplified pulses into current steering network 38. Current steering network 38 may typically comprise an H-bridge arrangement of switching transistor such as that used in motor drivers. The current steering network 38 directs the stimulation pulses to source at any one of the electrodes 14, 16 and 18 and to sink at any other of the electrodes depending upon the mode / intensity selected by the user 20. Stimulation pulses are returned to the power ground 46 of the system. This provides a route through which the electrical current may flow (through the user's abdominal muscles). It should be noted that although the previously described steering network is preferable, alternative arrangements are possible using, for example, transformers or relays.

When the pulse signal reaches, for example, the left electrode 14 a potential is created between electrode 14 and 18. The only route for the current to flow between electrode 14 and 18 is through the muscles of the user's abdomen and it is this flow of current which causes the abdominal muscles to contract (when current flows) and relax (when current does not flow). The cycle of contraction and relaxation can typically be varied as desired and is typically in the region of around 6 cycles per minute. A similar effect occurs on the abdominal muscles on the right hand side of the user's abdominal muscles when the pulse signal is sent to the right electrode 16.

This allows the user's abdominal muscles to be stimulated throughout the exercise regardless of the activity undertaken.

As the user 20 is exercising, the activity of his/her heart will increase (relative to resting heart activity) and it is desirable for the user to be able to monitor this. In the present embodiment, this is achieved using the remote sensor 24 in conjunction with the left abdominal electrode 14. It should be noted that, when used in this way, the electrode 14 may be regarded as a sensor 14 since it is used in a sensing mode. Similarly, right electrode 16 and central electrode 18 may be regarded as sensors if they are used in a sensing mode.

The location of the remote sensor 24 allows the activity of the heart to be monitored at the thoracic (chest) region. The measurement signal of this activity of the heart is commonly referred to as an "electrocardiogram"(ECG).

The electrical potential difference between sensor 24 and electrode 14 will be relatively high due to the relatively large difference in distances between the sensor 24 and electrode 14 from the source (the heart). The location of the left electrode 14 allows the activity of the heart to be monitored at the abdominal region. The signal detected by the electrode 14 will be relatively weak when compared to that detected at the sensor 24. The signal at electrode 14 is weak since the electric dipole which produces the signal on the user's chest is located in the thoracic region and is significantly attenuated in the lower abdominal region.

The potential difference between remote sensor 24 and electrode 14 is a dynamic ECG signal representative of the cardiac cycle. The resulting ECG signal can be directly related to the heart rate of the user 20 by inputting the ECG signal into differential amplifier 40, which amplifies the signal, and then passing the amplified signal into band pass filter 42 which extracts the heart rate as a series of pulses. This signal is then passed to the micro-controller 32, via threshold detector 44, which displays the heart rate to the user on display 48. In this regard, the threshold detector 44 is preferably an automatic threshold level detector which automatically adjusts the threshold level in proportion to the average signal amplitude over a sample period, typically of a few seconds. This allows a useful signal to be recovered that is not drowned out by the electrical noise created by movement of the user 20 during the exercise routine.

In addition to using the left electrode 14 in conjunction with the remote sensor 24, the right electrode 16 and central electrode 18 can also be used as a reference electrode to reduce interference and inaccuracies in the measured ECG. This is particularly useful in reducing movement artefact which occurs due to the vertical accelerations of the body caused by walking and running.

It should be noted that although described separately above, when both the muscle stimulation mode and the heart rate mode are activated by the user 20, the micro-controller 32 rapidly switches between providing the muscle stimulation and monitoring the heart rate. This has the effect of apparently doing both tasks simultaneously. In this regard, the heart rate signal monitored by the micro-processor 32 should not be affected by the electrical disturbance created by the muscle stimulation electrodes since it is timed to only monitor heart rate when no muscle stimulation is taking place.

This enables the user to monitor their heart rate in order to keep within a specific exercise zone. Such an exercise zone may be in the region of 60% to 70% of the theoretical maximum heart rate for a person of that age and fitness level. This allows the user to increase their work rate if the heart rate falls below the minimum recommended threshold and to decrease it if their heart rate goes above the maximum recommended threshold.

Although the micro-controller 32 is programmed to only monitor heart rate when the stimulation pulse is not being applied, the stimulation pulse will attempt to pass through the remote sensor 24 and any of the inactive electrodes back towards the differential amplifier 40. This would create an unintended leakage current and possibly damage the input of the amplifier 40 and protection against this should therefore be provided.

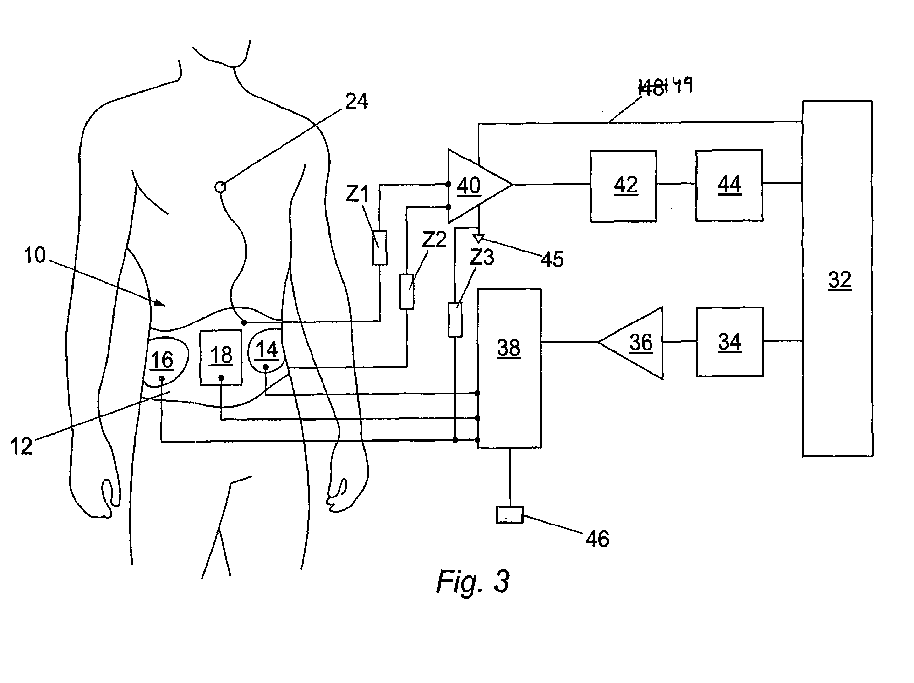

As shown in Fig. 3, protection against the above mentioned is provided by placing limiting resistors Z1 and Z2 before the differential amplifier 40 and resistor Z3 across the steering network output and the signal ground. These may be used in conjunction with voltage limiting devices such as zener diodes to protect against excessive voltages being applied to the amplifier 40 input. Choosing the value of resistors Z1, Z2 and Z3 involves considering the specific nature of the present system as discussed subsequently.

As will be appreciated by the reader, the stimulation provided by the electrodes on the band 12 causes a large electrical disturbance on the surface of the user's body and this must be accounted for in the detection of the heart rate in order to maintain accuracy.

Bio-amplifiers (amplifiers which amplify biological signals) are often AC coupled at their inputs since they must reject the DC potential (known as "half-cell potential") component which occurs at the sensing electrodes. In order to achieve this without compromising low frequency response, a high value capacitor, often greater than 1μF, is normally used. The problem with this solution is that high value capacitance coupled with the high resistance required to protect the amplifier 40 leads to long time constants for amplifier recovery following application of a stimulation pulse. This is because the AC coupling capacitor charges up during the stimulation pulse and must discharge before the amplifier 40 can continue operation. A compromise must therefore be reached, where a reasonable time constant is provided. This typically lies in the region somewhere between 0.25 seconds and 2 seconds. Placing these components before the amplifier 40 has a similar effect to preceding the amplifier 40 by a first order high pass filter. This results in a loss of some low frequency information in the ECG signal detected; however, this is acceptable in the present application since ECG signal fidelity is not particularly important and the principle measure of interest here is heart rate. The heart rate signal will be substantially unaffected by such a loss in ECG fidelity caused by the high pass filter. Taking into consideration these factor, a suitable time constraint is provided using, for example, 5MΩ of amplifier input resistance and 47nF of AC coupling capacitor.

A blanking signal represented by line 49 can also be selectively applied to the amplifier 40 to switch it off during stimulation pulses. A similar blanking signal (not shown) can be used to disable the threshold detector 44.

As well as protecting components of the apparatus 10 from damage it is important to ensure that the user 20 is protected from excessive and wayward electrical pulses. For this reason, safety standards specify that electrodes (such as sensor 24) which are intended to only detect electrical signals (ECG signals) must not be allowed to *deliver* stimulation pulses. Such unintentional current is know as an auxiliary current and, if allowed, could be dangerous to the user due to the sensor 24 proximity to the user's heart.

Various switches such as transistors and relays may be placed in the control circuit in order to both protect components of the circuit and to protect the user 20; however, such components can fail in extreme conditions and it is therefore necessary to ensure further protection to ensure that the device "fails safe".

Preventing leakage of auxiliary current requires that the input resistance to the amplifier 40 is both high and capable of withstanding high input voltages. The amplifier 40 will have an inherent resistance; however, it is not sufficient to rely on this resistance since it may break down with high voltages. It is therefore safest to place an additional high resistance resistor, in the order of 100kΩ, in series on each lead connecting the electrodes 14, 16 and the sensor 24 to the micro-controller 32. These resistors are represented on Fig.3 by Z1, Z2 and Z3 respectively.

Accordingly, the invention described provides exercise equipment which allows the user to actively monitor their heart rate and muscle stimulation to individual preference without the risk of being subjected to uncontrolled electrical pulses.

The invention therefore allows the user to simultaneously perform two different forms of exercise at any one time; namely, Electrical Muscle Stimulation and cardiovascular exercise with heart rate monitoring. Electrical Muscle Stimulation does not, by itself, create a significant load to the cardiovascular system and therefore has little or no exercise benefit to that system. It does however load and thereby promote performance improvement in the target muscle.

In cardiovascular exercise, the value of an exercise session can be estimated by the amount of time the user spends at or above the target heart rate. For resistance training on the other hand, time is not important and instead it is the number of repetitions of muscle contraction under load that is important.

The present invention provides for the calculation and display of an exercise Quality Factor which is based on the amount of time the user spends at or above the target heart rate and the number of stimulated muscle contractions completed.

The resultant Quality Factor is displayed to the user (either on the display device or by an audio device) and is updated as the user exercises. The Quality Factor is calculated according to the following equation:-

$$QF = k_1 T + k_2 (c \times c_i)$$

Where QF is the Quality Factor, $k_1$ is a weighting and scaling component for the cardiovascular component of the exercise, T is the time in minutes of exercise at or above a target heart rate, $k_2$ is a weighting and scaling factor for the electrical stimulation components of the exercise
c is the number of contractions per minute (set by the user) and $c_i$ is contraction intensity (set by the user).

The contraction intensity is changed by altering the flow of current between the electrodes and can be altered by the user to a desired level, or as in the following example, may be set to 1.

A typical value for k1 would be k1=1/15 and for k2 would be k2= 1/180 although obviously other values could also be chosen, depending on the precision of the display device and the relative importance of each component of the exercise. With the above values, a 60 minute walking or jogging exercise at the target heart rate, with 3 maximal muscle contractions per minute would yield a Quality Factor of 5 according to the following:-

$$QF = \frac{1}{15} \times 60 + \frac{1}{180} (3 \times 60 \times 1) = 5$$

The combination of Electrical Muscle Stimulation and cardiac monitoring in the present invention also has the advantage of allowing the electrical stimulus provided to desired muscles to coincide with a defined point in the cardiac cycle. For example, the delivery of the stimulus may be timed to occur a fixed time before the R wave of the ECG. The extent of the time delay can be adjusted to improve the efficacy of the assisted venous return.

Modifications and improvements may be made to the foregoing without departing from the scope of the invention, for example:

The micro-controller 32 can be programmed to provide a personalised muscle stimulation sequence which may be saved in the memory of the micro-controller 32. This could also automatically adapt the stimulation cycle to take account of the heart rate detected, that is, if the detected heart rate is high, the stimulation provided by the band 12 would automatically increase in order to increase the intensity of the overall exercise and vice versa. Alternatively, the micro-controller 32 may be programmed to decrease the intensity of the stimulation provided by the electrodes on the band 12 as the heart rate increases in order to focus training on the cardiovascular exercise as opposed to the abdominal stimulation and vice versa.

The abdominal band 12 could be replaced by or complemented with other groups of stimulation electrodes such as thigh and buttock electrodes.

The control unit 30 may be incorporated into a wristwatch or other separate unit which uses a wireless communication to transmit and receive information to and from the base unit 28.

The invention employs a sensor 24 located close to the user's heart in conjunction with a sensor 14 located at the abdominal region (provided for instance by electrode 14) in order to detect the electrical potential there between (and hence the heart rate of the user).

Although, primarily described in relation to muscle stimulation, the nerve stimulation apparatus described may alternatively be used for stimulation of nerves for pain relief or massage.

## Claims

1. Nerve stimulation apparatus (10) comprising:

    a control unit (30);
    a first muscle stimulation electrode (14) for attachment to an abdominal and/or thigh and/or buttock region of a user's body;
    a second muscle stimulation electrode (16) for attachment to an abdominal and/or thigh and/or buttock region of the user's body, wherein the control unit (30) is connected to said first and second electrodes (14,16) and is adapted to impart an electrical current between said first and second electrodes (14,16);
    a first sensor (24); and
    a second sensor (14) provided by said first muscle stimulation electrode (14), wherein the control unit (30) is connected to said first and second sensors (14,24) and is adapted to detect an electrical signal differential between said first and second sensors (14,24),
    **characterised in that** the apparatus (10) further comprises a band (12) adapted to be placed adjacent the abdominal and/or thigh and/or buttock muscles on which said first and second electrodes (14,16) are provided; and **in that**
    the first sensor (24) is adapted to be attached to a thoracic region of the user's body away from said band (12).

2. Nerve stimulation apparatus according to claim 1, further comprising a third sensor provided by said second muscle stimulation electrode (16).

3. Nerve stimulation apparatus according to any preceding claim, further comprising connection means (22) connecting the first sensor (24) to said control unit (30).

4. Nerve stimulation apparatus according to any preceding claim, further comprising a third muscle stimulation electrode (18) provided on said band (12) between said first and second muscle stimulation electrodes (14,16).

5. Nerve stimulation apparatus according to claim 4, wherein said third muscle stimulation electrode (18) is connected to the control unit (30), which is adapted to use the third muscle stimulation electrode (18) as a common node and pass an electrical current between said third muscle stimulation electrode (18) and said first muscle stimulation electrode (14) and selectively between said third muscle stimulation electrode (18) and said second muscle stimulation electrode (16).

6. Nerve stimulation apparatus according to any preceding claim, wherein said control unit (30) is adapted to alternate between a stimulation mode where selected nerves and hence selected muscles are stimulated and a monitoring mode where electrical activity of the user's heart is monitored, wherein when the stimulating mode is in operation, the monitoring mode is deactivated.

7. Nerve stimulation apparatus according to claim 6, wherein the monitoring mode is deactivated by a blanking signal selectively emitted from said control unit (30) when said stimulating mode is in operation.

8. Nerve stimulation apparatus according to claim 6 or 7, wherein said control unit (30) is provided with safety means to substantially prevent electrical current produced as a result of the stimulation mode from entering each sensor (14,24).

9. Nerve stimulation apparatus according to claim 8, wherein the safety means is provided by an impedance.

10. Nerve stimulation apparatus according to either of claims 8 or 9, wherein said control unit (30) is provided with further safety means to substantially prevent electrical current being imparted from each sensor (14,24) into the user's body.

11. Nerve stimulation apparatus according to claim 10, wherein the further safety means is an impedance provided between said control unit (30) and each sensor (14,24).

12. Nerve stimulation apparatus according to any preceding claim, wherein said control unit (30) comprises a processor (32) adapted to provide an analogue pulse signal, and a converter (34) adapted to convert the analogue pulse signal into a digital pulse sequence.

13. Nerve stimulation apparatus according to claim 12, wherein said control unit (30) further comprises a pulse amplifier (36) adapted to amplify said digital pulse sequence.

14. Nerve stimulation apparatus according to claim 13, wherein said control unit (30) further comprises an H-bridge arrangement of switching transistors to provide a current steering network (38) adapted to selectively direct said amplified digital pulse sequence to the or each muscle stimulation electrode (14,16,18).

15. Nerve stimulation apparatus according to claim 14, wherein said control unit (30) further comprises a differential amplifier (40) adapted to amplify the signal obtained from the sensors (14,24).

16. Nerve stimulation apparatus according to claim 15, wherein said control unit (30) further comprises a band pass filter (42) adapted to derive, as a series of pulses, the user's heart rate from said amplified sensor signal.

17. Nerve stimulation apparatus according to claim 16, wherein said control unit (30) further comprises a threshold detector (44) adapted to filter electrical noise detected by the sensors (14,24).

18. Nerve stimulation apparatus according to claim 17, wherein said threshold detector (44) is adapted to automatically adjust a threshold level depending upon an average signal amplitude detected over a given sample period.

19. Nerve stimulation apparatus according to any preceding claim, wherein the apparatus further comprises a display unit (48) in communication with said control unit (30), the display unit (48) being capable of displaying data to the user such as heart rate, duration of exercise.

20. Nerve stimulation apparatus according to any preceding claim further comprising a command unit in communication with said control unit (30), the command unit being adapted to allow the user to modify variables such as an intensity of stimulation provided by said electrodes and an intended exercise duration.

21. Nerve stimulation apparatus according to claims 19 and 20, wherein the display unit (48) and the command unit are integrated into a module in communication with said control unit (30) and adapted to be hand held or located on a wristwatch.

22. Nerve stimulation apparatus according to any preceding claim, wherein the control unit (30) is adapted to provide the user with a Quality Factor which represents the level of benefit from the exercise and is dependent upon the level of exertion during exercise, wherein the control unit (30) is further adapted to calculate the Quality Factor according to the following equation:

$$QF = k_1 T + k_2(c \times c_i)$$

wherein
QF is the Quality Factor,
$k_1$ is a weighting and scaling component for a cardiovascular component of the exercise,
T is a time of exercise at or above a target heart rate,
$k_2$ is a weighting and scaling factor for electrical stimulation components of the exercise,
c is the number of contractions, and
$c_i$ is contraction intensity.

**Patentansprüche**

1. Vorrichtung zur Nervenstimulation (10), die Folgendes beinhaltet:

    eine Steuereinheit (30);
    eine erste Muskelstimulationselektrode (14) zur Anbringung an einem Unterleibs- und/oder Oberschenkel- und/oder Gesäßbackenbereich des Körpers eines Benutzers;
    eine zweite Muskelstimulationselektrode (16) zur Anbringung an einem Unterleibs- und/oder Oberschenkel- und/oder Gesäßbackenbereich des Körpers des Benutzers,
    wobei die Steuereinheit (30) mit der ersten und zweiten Elektrode (14, 16) verbunden ist und angepasst ist, um einen elektrischen Strom zwischen der ersten und zweiten Elektrode (14, 16) zu übermitteln;
    einen ersten Sensor (24); und
    einen durch die erste Muskelstimulationselektrode (14) bereitgestellten zweiten Sensor (14), wobei die Steuereinheit (30) mit dem ersten und zweiten Sensor (14, 24) verbunden ist und angepasst ist, um eine Differenz des elektrischen Signals zwischen dem ersten und zweiten Sensor (14, 24) zu erkennen, **dadurch gekennzeichnet, dass** die Vorrichtung (10) ferner einen Gurt (12) beinhaltet, der angepasst ist, um angrenzend an die Unterleibs- und/oder Oberschenkel- und/oder Gesäßbackenmuskeln positioniert zu werden, auf dem die erste und zweite Elektrode (14, 16) bereitgestellt sind; und **dadurch**, dass
der erste Sensor (24) angepasst ist, um an einem Brustbereich des Körpers des Benutzers entfernt von dem Gurt (12) angebracht zu werden.

2. Vorrichtung zur Nervenstimulation gemäß Anspruch 1, die ferner einen durch die zweite Muskelstimulationselektrode (16) bereitgestellten dritten Sensor beinhaltet.

3. Vorrichtung zur Nervenstimulation gemäß einem der vorhergehenden Ansprüche, die ferner ein Verbindungsmittel (22) beinhaltet, das den ersten Sensor (24) mit der Steuereinheit (30) verbindet.

4. Vorrichtung zur Nervenstimulation gemäß einem der vorhergehenden Ansprüche, die ferner eine dritte Muskelstimulationselektrode (18), die zwischen der ersten und zweiten Muskelstimulationselektrode (14, 16) auf dem Gurt (12) bereitgestellt ist, beinhaltet.

5. Vorrichtung zur Nervenstimulation gemäß Anspruch 4, wobei die dritte Muskelstimulationselektrode (18) mit der Steuereinheit (30) verbunden ist, die angepasst ist, um die dritte Muskelstimulationselektrode (18) als einen gemeinsamen Knoten zu verwenden und einen elektrischen Strom zwischen der dritten Muskelstimulationselektrode (18) und der ersten Muskelstimulationselektrode (14) und selektiv zwischen der dritten Muskelstimulationselektrode (18) und der zweiten Muskelstimulationselektrode (16) zu leiten.

6. Vorrichtung zur Nervenstimulation gemäß einem der vorhergehenden Ansprüche, wobei die Steuereinheit (30) angepasst ist, um zwischen einem Stimulationsmodus, in dem ausgewählte Nerven und folglich ausgewählte Muskeln stimuliert werden, und einem überwachenden Modus, in dem die elektrische Aktivität des Herzens des Benutzers überwacht wird, zu alternieren, wobei, wenn der stimulierende Modus in Betrieb ist, der überwachende Modus deaktiviert ist.

7. Vorrichtung zur Nervenstimulation gemäß Anspruch 6, wobei der überwachende Modus durch ein Austastsignal, das selektiv von der Steuereinheit (30) emittiert wird, wenn der stimulierende Modus in Betrieb ist, deaktiviert wird.

8. Vorrichtung zur Nervenstimulation gemäß Anspruch 6 oder 7, wobei die Steuereinheit (30) mit einem Sicherheitsmittel versehen ist, um im Wesentlichen zu verhindern, dass elektrischer Strom, der als ein Resultat des Stimulationsmodus produziert wird, in jeden Sensor (14, 24) eintritt.

9. Vorrichtung zur Nervenstimulation gemäß Anspruch 8, wobei das Sicherheitsmittel durch eine Impedanz bereitgestellt wird.

10. Vorrichtung zur Nervenstimulation gemäß einem der Ansprüche 8 oder 9, wobei die Steuereinheit (30) mit einem weiteren Sicherheitsmittel versehen ist, um im Wesentlichen zu verhindern, dass elektrischer Strom von jedem Sensor (14, 24) in den Körper des Benutzers übermittelt wird.

11. Vorrichtung zur Nervenstimulation gemäß Anspruch 10, wobei das weitere Sicherheitsmittel eine zwischen der Steuereinheit (30) und jedem Sensor (14, 24) bereitgestellte Impedanz ist.

12. Vorrichtung zur Nervenstimulation gemäß einem der vorhergehenden Ansprüche, wobei die Steuereinheit (30) einen Prozessor (32), der angepasst ist, um ein analoges Impulssignal bereitzustellen, und einen Wandler (34), der angepasst ist, um das analoge Impulssignal in eine digitale Impulsfolge umzuwandeln, beinhaltet.

13. Vorrichtung zur Nervenstimulation gemäß Anspruch 12, wobei die Steuereinheit (30) ferner einen Impulsverstärker (36) beinhaltet, der angepasst ist, um die digitale Impulsfolge zu verstärken.

14. Vorrichtung zur Nervenstimulation gemäß Anspruch 13, wobei die Steuereinheit (30) ferner eine B-Brückenanordnung aus Schalttransistoren beinhaltet, um ein Stromsteuerungsnetz (38) bereitzustellen, angepasst, um selektiv die verstärkte digitale Impulsfolge zu der oder jeder Muskelstimulationselektrode (14, 16, 18) zu lenken.

15. Vorrichtung zur Nervenstimulation gemäß Anspruch 14, wobei die Steuereinheit (30) ferner einen Differenzverstärker (40) beinhaltet, der angepasst ist, um das von den Sensoren (14, 24) erhaltene Signal zu verstärken.

16. Vorrichtung zur Nervenstimulation gemäß Anspruch 15, wobei die Steuereinheit (30) ferner einen Bandpassfilter (42) beinhaltet, der angepasst ist, um die Herzfrequenz des Benutzers als eine Serie von Impulsen aus dem verstärkten Sensorsignal zu erlangen.

17. Vorrichtung zur Nervenstimulation gemäß Anspruch 16, wobei die Steuereinheit (30) ferner einen Schwellendetektor (44) beinhaltet, der angepasst ist, um durch die Sensoren (14, 24) erkanntes Elektrorauschen zu filtern.

18. Vorrichtung zur Nervenstimulation gemäß Anspruch 17, wobei der Schwellendetektor (44) angepasst ist, um einen Schwellenwert, abhängig von einer durchschnittlichen Signalamplitude, erkannt während einer gegebenen Abtastperiode, automatisch anzugleichen.

19. Vorrichtung zur Nervenstimulation gemäß einem der vorhergehenden Ansprüche, wobei die Vorrichtung ferner eine Anzeigeeinheit (48) in Verbindung mit der Steuereinheit (30) beinhaltet, wobei die Anzeigeeinheit (48) imstande ist, dem Benutzer Daten wie etwa Herzfrequenz, Dauer der Betätigung anzuzeigen.

20. Vorrichtung zur Nervenstimulation gemäß einem der vorhergehenden Ansprüche, die ferner eine Befehlseinheit in Verbindung mit der Steuereinheit (30) beinhaltet, wobei die Befehlseinheit angepasst ist, um dem Benutzer zu ermöglichen, Variablen wie etwa eine Intensität der durch die Elektroden bereitgestellten Stimulation und eine beabsichtigte Betätigungsdauer zu modifizieren.

21. Vorrichtung zur Nervenstimulation gemäß den Ansprüchen 19 und 20, wobei die Anzeigeeinheit (48) und die Befehlseinheit in ein Modul in Verbindung mit der Steuereinheit (30) integriert sind und angepasst sind, um in der Hand gehalten zu werden oder auf einer Armbanduhr untergebracht zu sein.

22. Vorrichtung zur Nervenstimulation gemäß einem der vorhergehenden Ansprüche, wobei die Steuereinheit (30) angepasst ist, dem Benutzer einen Qualitätsfaktor bereitzustellen, der den Grad an Nutzen von der Betätigung darstellt und von dem Grad an Anstrengung während der Betätigung abhängt, wobei die Steuereinheit (30) ferner angepasst ist, um den Qualitätsfaktor gemäß der folgenden Gleichung zu berechnen:

$$QF = k_1 T + k_2(c \times c_i)$$

wobei
QF der Qualitätsfaktor ist,
$k_1$ eine Gewichtungs- und Normierungskomponente für eine kardiovaskuläre Komponente der Betätigung ist,
T eine Zeit der Betätigung bei oder über einer Zielherzfrequenz ist,
$k_2$ ein Gewichtungs- und Normierungsfaktor für Komponenten der elektrischen Stimulation der Betätigung ist,
c die Anzahl an Kontraktionen ist und
$c_i$ die Kontraktionsintensität ist.

**Revendications**

1. Appareil de stimulation nerveuse (10) comprenant :

   une unité de contrôle (30) ;
   une première électrode de stimulation musculaire (14) destinée à être attachée à une région abdominale et / ou crurale et / ou fessière du corps d'un utilisateur ;
   une deuxième électrode de stimulation musculaire (16) destinée à être attachée à une région abdominale et / ou crurale et / ou fessière du corps de l'utilisateur, dans lequel l'unité de contrôle (30) est connectée auxdites première et deuxième électrodes (14, 16) et est adaptée pour communiquer un courant électrique entre lesdites première et deuxième électrodes (14, 16) ;
   un premier capteur (24) ; et
   un deuxième capteur (14) fourni par ladite première électrode de stimulation musculaire (14), dans lequel l'unité de contrôle (30) est connectée auxdits premier et deuxième capteurs (14, 24) et est adaptée pour détecter un différentiel de signal électrique entre lesdits premier et deuxième capteurs (14, 24), **caractérisé en ce que** l'appareil (10) comprend de plus une bande (12) adaptée pour être placée de façon adjacente aux muscles abdominaux et / ou cruraux et / ou fessiers sur lesquels lesdites première et deuxième électrodes (14, 16) sont fournies ; et **en ce que**
   le premier capteur (24) est adapté pour être attaché à une région thoracique du corps de l'utilisateur à distance de ladite bande (12).

2. Appareil de stimulation nerveuse selon la revendication 1, comprenant de plus un troisième capteur fourni par ladite deuxième électrode de stimulation musculaire (16).

3. Appareil de stimulation nerveuse selon n'importe quelle revendication précédente, comprenant de plus un moyen de connexion (22) connectant le premier capteur (24) à ladite unité de contrôle (30).

4. Appareil de stimulation nerveuse selon n'importe quelle revendication précédente, comprenant de plus une troisième électrode de stimulation musculaire (18) fournie sur ladite bande (12) entre lesdites première et deuxième électrodes de stimulation musculaire (14, 16).

5. Appareil de stimulation nerveuse selon la revendication 4, dans lequel ladite troisième électrode de stimulation musculaire (18) est connectée à l'unité de contrôle (30), laquelle est adaptée pour utiliser la troisième électrode de stimulation musculaire (18) comme noeud commun et faire passer un courant électrique entre ladite troisième électrode de stimulation musculaire (18) et ladite première électrode de stimulation musculaire (14) et de façon sélective entre ladite troisième électrode de stimulation musculaire (18) et ladite deuxième électrode de stimulation musculaire (16).

6. Appareil de stimulation nerveuse selon n'importe quelle revendication précédente, dans lequel ladite unité de contrôle (30) est adaptée pour alterner entre un mode stimulation où des nerfs sélectionnés et donc des muscles sélectionnés sont stimulés et un mode surveillance où l'activité électrique du coeur de l'utilisateur est surveillée, dans lequel, lorsque le mode stimulant est en opération, le mode surveillance est désactivé.

7. Appareil de stimulation nerveuse selon la revendication 6, dans lequel le mode surveillance est désactivé par un signal de suppression émis de façon sélective par ladite unité de contrôle (30) lorsque ledit mode stimulant est en opération.

8. Appareil de stimulation nerveuse selon la revendication 6 ou la revendication 7, dans lequel ladite unité de contrôle (30) est pourvue d'un moyen de sécurité pour empêcher substantiellement que du courant électrique produit en résultat du mode stimulation n'entre dans chaque capteur (14, 24).

9. Appareil de stimulation nerveuse selon la revendication 8, dans lequel le moyen de sécurité est fourni par une impédance.

10. Appareil de stimulation nerveuse selon l'une ou l'autre des revendications 8 et 9, dans lequel ladite unité de contrôle (30) est pourvue d'un moyen de sécurité supplémentaire pour empêcher substantiellement que du courant électrique communiqué depuis chaque capteur (14, 24) n'entre dans le corps de l'utilisateur.

11. Appareil de stimulation nerveuse selon la revendication 10, dans lequel le moyen de sécurité supplémentaire est une impédance fournie entre ladite unité de contrôle (30) et chaque capteur (14, 24).

12. Appareil de stimulation nerveuse selon n'importe quelle revendication précédente, dans lequel ladite unité de contrôle (30) comprend un processeur (32) adapté pour fournir un signal à impulsions analogiques, et un convertisseur (34) adapté pour convertir le signal à impulsions analogiques en une séquence d'impulsions numériques.

13. Appareil de stimulation nerveuse selon la revendication 12, dans lequel ladite unité de contrôle (30) comprend de plus un amplificateur d'impulsions (36) adapté pour amplifier ladite séquence d'impulsions numériques.

14. Appareil de stimulation nerveuse selon la revendication 13, dans lequel ladite unité de contrôle (30) comprend de plus un arrangement de type pont en H de transistors de commutation pour fournir un réseau d'aiguillage de courant (38) adapté pour diriger de façon sélective ladite séquence d'impulsions numériques amplifiées vers la ou chaque électrode de stimulation musculaire (14, 16, 18).

15. Appareil de stimulation nerveuse selon la revendication 14, dans lequel ladite unité de contrôle (30) comprend de plus un amplificateur différentiel (40) adapté pour amplifier le signal obtenu des capteurs (14, 24).

16. Appareil de stimulation nerveuse selon la revendication 15, dans lequel ladite unité de contrôle (30) comprend de plus un filtre passe-bande (42) adapté pour dériver, sous forme d'une série d'impulsions, la fréquence cardiaque de l'utilisateur dudit signal de capteur amplifié.

17. Appareil de stimulation nerveuse selon la revendication 16, dans lequel ladite unité de contrôle (30) comprend de plus un détecteur à seuil (44) adapté pour filtrer du bruit électrique détecté par les capteurs (14, 24).

18. Appareil de stimulation nerveuse selon la revendication 17, dans lequel ledit détecteur à seuil (44) est adapté pour ajuster de façon automatique un niveau de seuil en fonction d'une amplitude de signal moyenne détectée sur une période échantillon donnée.

19. Appareil de stimulation nerveuse selon n'importe quelle revendication précédente, dans lequel l'appareil comprend de plus une unité d'affichage (48) en communication avec ladite unité de contrôle (30), l'unité d'affichage (48) étant capable d'afficher des données à l'intention de l'utilisateur telles que la fréquence cardiaque, la durée d'exercice.

20. Appareil de stimulation nerveuse selon n'importe quelle revendication précédente comprenant de plus une unité de commande en communication avec ladite unité de contrôle (30), l'unité de commande étant adaptée pour permettre à l'utilisateur de modifier des variables telles qu'une intensité de stimulation fournie par lesdites électrodes et une durée d'exercice envisagée.

21. Appareil de stimulation nerveuse selon les revendications 19 et 20, dans lequel l'unité d'affichage (48) et l'unité de commande sont intégrées dans un module en communication avec ladite unité de contrôle (30) et adaptées pour être tenues à la main ou placées sur un bracelet-montre.

22. Appareil de stimulation nerveuse selon n'importe quelle revendication précédente, dans lequel l'unité de contrôle (30) est adaptée pour fournir à l'utilisateur un Facteur de Qualité qui représente le niveau de bénéfice tiré de l'exercice et qui dépend du niveau d'effort durant l'exercice, dans lequel l'unité de contrôle (30) est de plus adaptée pour calculer le Facteur de Qualité selon l'équation suivante :

$$QF = k_1 T + k_2 (c \times c_i)$$

dans laquelle
QF est le Facteur de Qualité,
$k_1$ est une composante de pondération et de mise à l'échelle pour une composante cardiovasculaire de l'exercice,
T est un temps d'exercice à ou au-dessus d'une fréquence cardiaque cible,
$k_2$ est un facteur de pondération et de mise à l'échelle pour des composantes de stimulation électrique de l'exercice,
c est le nombre de contractions, et
$c_i$ est l'intensité des contractions.

*Fig. 1*

*Fig. 2*

EP 1 871 467 B1

*Fig. 3*

EP 1 871 467 B1

*Fig. 4*

EP 1 871 467 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 2374288 A